# EUROPEAN PATENT SPECIFICATION

(11) **EP 2 515 874 B1**
(45) Date of publication and mention of the grant of the patent: **20.08.2014**
(21) Application number: 10803440.6
(22) Date of filing: 22.12.2010
(51) Int. Cl.: A61K 9/00, A61K 9/14, A61K 31/593

(54) **CALCIPOTRIOL MONOHYDRATE NANOCRYSTALS**
CALCIPOTRIOL MONOHYDRAT NANOKRISTALLE
NANOCRISTAUX DE MONOHYDRATE DE CALCIPOTRIOL

(30) Priority: 22.12.2009 WO PCT/DK2009/000267; 07.01.2010 US 293091 P
(43) Date of publication of application: 31.10.2012
(73) Proprietor: Leo Pharma A/S, 2750 Ballerup (DK)
(72) Inventor: PETERSSON, Karsten, DK-2750 Ballerup (DK)
(74) Representative: Thalsoe-Madsen, Kine Birgit
(86) International application number: PCT/DK2010/000183
(87) International publication number: WO 2011/076208

(56) References cited:
- WO-A1-94/15912
- WO-A1-2008/058755
- US-A1- 2005 281 886
- US-A1- 2006 292 080
- "32300 Psorcutan Beta 50 Mikrogramm/g + 0,5 mg/g Salbe" In: "Rote Liste 2009", 1 January 2009 (2009-01-01), Rote Liste Service GmbH, XP55015790, ISBN: 978-3-93-919230-5 Monorgaphy 32300

## Description

### FIELD OF INVENTION

The present invention relates to calcipotriol monohydrate in the form of nanocrystals and the inclusion of the nanocrystals in a pharmaceutical composition intended for use in the prevention or treatment of dermal diseases and conditions.

### BACKGROUND OF THE INVENTION

Psoriasis is a chronic inflammatory skin disease that manifests as erythematous, dry, scaling plaques resulting from hyperkeratosis. The plaques are most often found on the elbows, knees and scalp, though more extensive lesions may appear on other parts of the body, notably the lumbosacral region. The most common treatment of mild to moderate psoriasis involves topical application of a composition containing a corticosteroid as the active ingredient. While efficacious, corticosteroids have the disadvantage of a number of adverse effects such as skin atrophy, striae, acneiform eruptions, perioral dermatitis, overgrowth of skin fungus and bacteria, hypopigmentation of pigmented skin and rosacea.

For many years, however, an advantageous non-steroidal treatment of psoriasis has consisted in topical treatment with the vitamin D analogue compound, calcipotriol, formulated in an ointment composition (marketed as Daivonex^{®} or Dovonex^{®} ointment by LEO Pharma) in which the calcipotriol is present in solution or a cream composition (marketed as Daivonex^{®} or Dovonex^{®} cream by LEO Pharma) in which the calcipotriol is present as a suspension of microparticles. The solvent in the ointment composition is propylene glycol which has the advantage of enhancing penetration of the active ingredient into the skin, leading to an improved efficacy, but which is also known to act as a skin irritant. Thus, it has been reported that the inclusion of propylene glycol in topical compositions frequently causes patients to develop contact dermatitis (one study reported a number of irritant reactions to propylene glycol of 12.5%, cf. M. Hannuksela et al., Contact Dermatitis 1, 1975, pp. 112-116), and the number of irritant reactions increases when propylene glycol is used in high concentrations (as reviewed by J. Catanzaro and J. Graham Smith, J. Am. Acad. Dermatol. 24, 1991, pp. 90-95). Due to the improved penetration of calcipotriol into the skin resulting, *inter alia,* from the presence of propylene glycol, Daivonex^{®} ointment has been found to be more efficacious in the treatment of psoriatic lesions than Daivonex^{®} cream, but has also caused skin irritation in a significant proportion of psoriasis patients.

### SUMMARY OF THE INVENTION

Human skin, in particular the outer layer, the stratum corneum, provides an effective barrier against penetration of microbial pathogens and toxic chemicals. While this property of skin is generally beneficial, it complicates the dermal administration of pharmaceuticals in that a large quantity, if not most, of the active ingredient applied on the skin of a patient suffering from a dermal disease may not penetrate into the viable layers of the skin where it exerts its activity. To ensure adequate penetration of the active ingredient to the dermis and epidermis, it is generally preferred to include the active ingredient in a dissolved state, typically in the presence of a solvent in the form of an alcohol, e.g. ethanol, or diol, e.g. propylene glycol. Propylene glycol is a well-known penetration enhancer, i.e. a substance which is capable of penetrating the stratum corneum and "draw" low-molecular components such as therapeutically active components in the vehicle into the epidermis. Propylene glycol may in itself give rise to significant skin irritation, and it is also capable of "drawing" low-molecular and potentially irritative components of the vehicle into the epidermis, leading to an overall irritative effect of conventional vehicles including propylene glycol. For this reason, the presence of propylene glycol as a solvent in compositions intended for the treatment of inflammatory skin diseases may exacerbate the inflammatory response.

In the research leading to the present invention, it was an object to provide a topical composition comprising calcipotriol as the active ingredient, which has skin penetration and biological activity properties comparable to those of Daivonex^{®} ointment, but which does not contain propylene glycol as the solvent.

It has surprisingly been found possible to prepare calcipotriol monohydrate in the form of nanocrystals which are chemically stable (i.e. not degraded into 24-epi calcipotriol or other degradation products) as unexpectedly no significant amounts of amorphous calcipotriol are formed as a result of high stress or impact forces or high temperatures during nanosizing. Furthermore, the nanocrystals are physically stable as no aggregation or crystal growth or change in crystal (polymorphic) form is observed in a suspension of the nanocrystals after preparation. The nanocrystals are readily formulated into topical cream and ointment compositions from which calcipotriol (monohydrate) may penetrate into viable layers of the skin (i.e. the dermis and epidermis) in amounts comparable to the penetration of calcipotriol from Daivonex^{®} ointment and result in similar or higher levels of biological activity (as determined by *in vitro* activation of a target gene) without resorting to the inclusion of a penetration enhancer such as propylene glycol which is a potential skin irritant.

Accordingly, in one aspect the present invention relates to a suspension of calcipotriol monohydrate in the form of nanocrystals of a particle size distribution in the range of 200-600 nm as determined by dynamic light scattering, the suspension further comprising an aqueous phase including a non-ionic, polymeric surfactant in an amount sufficient to prevent formation of aggregates and/or crystal growth of the calcipotriol monohydrate nanocrystals.

In another aspect, the application relates to a process for preparing calcipotriol monohydrate nanocrystals of a particle size distribution in the range of 200-600 nm as determined by dynamic light scattering, the process comprising the steps of
(a) diminuting crystalline calcipotriol monohydrate in an aqueous phase comprising non-ionic, polymeric surfactant in an amount in the range of from about 1% to about 5% by weight of said aqueous phase, resulting to the formation of microparticles with a particle size distribution in the range of about 5-20 µm and a mean particle size of about 10 µm;
(b) subjecting the suspension of step (a) to a first cycle of high pressure homogenization at a pressure of about 300-800 bar for a period of time sufficient to obtain about 15-40% of crystals of calcipotriol monohydrate with a particle size distribution in the range of 200-600 nm;
(c) subjecting the suspension of step (b) to a second cycle of high pressure homogenization at a pressure of about 800-1200 bar a period of time sufficient to obtain about 40-80% of crystals of calcipotriol monohydrate with a particle size distribution in the range of 200-600 nm;
(d) subjecting the suspension of step (c) to a third cycle of high pressure homogenization at a pressure of about 1200-1700 bar a period of time sufficient to obtain about 90% or more of crystals of calcipotriol monohydrate with a particle size distribution in the range of 200-600 nm; and
(e) optionally isolating the resulting nanocrystals of calcipotriol monohydrate from the aqueous phase.

In a further aspect, the invention relates to a pharmaceutical composition comprising the calcipotriol monohydrate nanocrystals described above and a pharmaceutically acceptable carrier.

In a still further aspect, the invention relates to the use of the composition comprising calcipotriol monohydrate nanocrystals or nanosuspension for the treatment of dermal diseases or conditions such as psoriasis, sebopsoriasis, pustulosis palmoplantaris, dermatitis, ichtyosis, rosacea or acne.

### BRIEF DESCRIPTION OF THE DRAWINGS

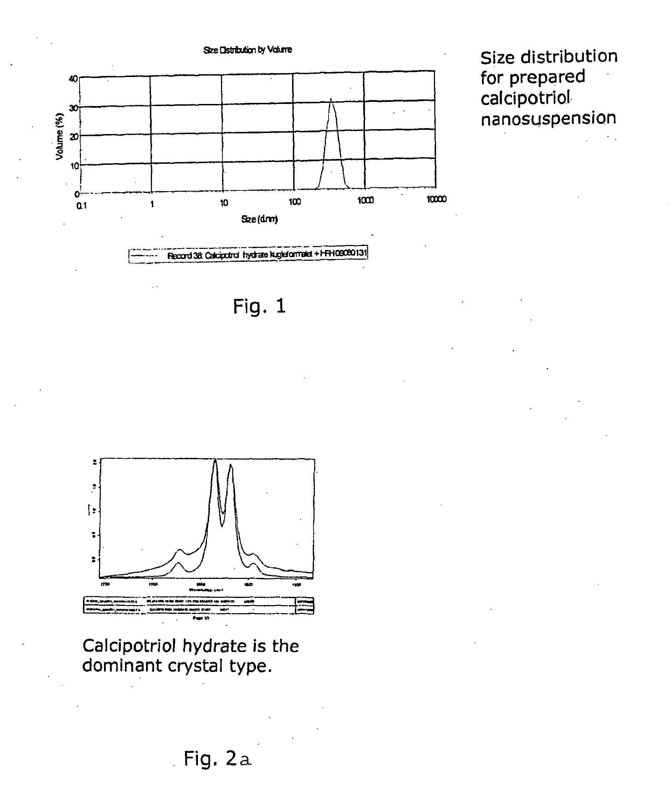

Fig. 1 is a graph showing the particle size distribution of the calcipotriol monohydrate nanocrystals prepared by the present process, as determined by dynamic light scattering.
Fig 2a is a graph comparing the Raman spectrum of a calcipotriol monohydrate nanosuspension in 2% poloxamer 188 with a Raman spectrum of calcipotriol monohydrate not subjected to nanosizing. The figure shows that the nanosizing process according to the invention does not result in any change in the crystal form of calcipotriol monohydrate.

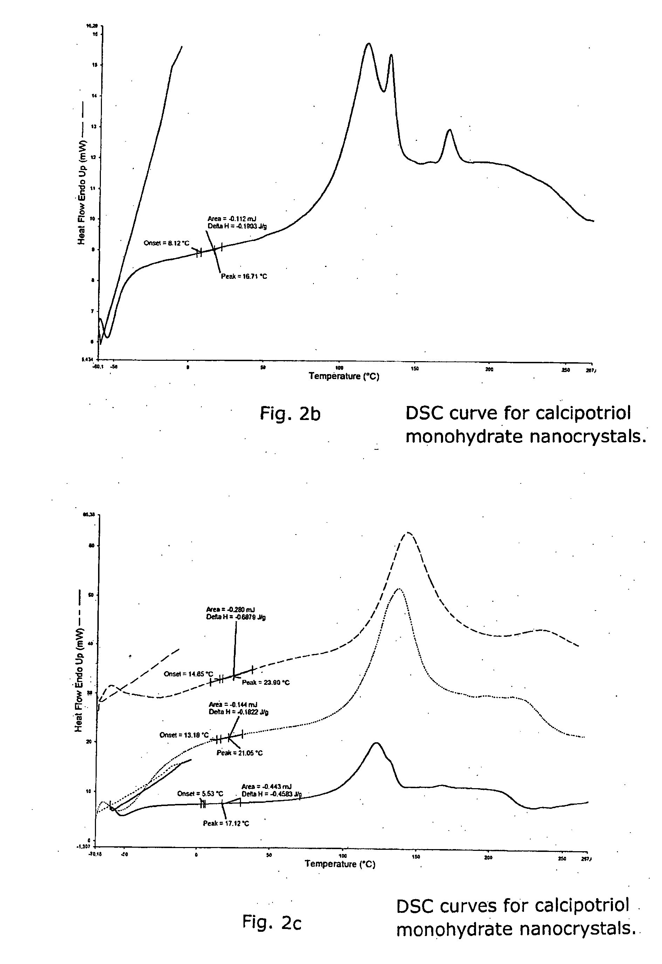

Fig. 2b and 2c are graphs showing the results of differential scanning calorimetry (DSC) analysis of two batches of calcipotriol monohydrate nanocrystals prepared by the present process. The DSC was conducted at 100°C/min (Fig. 2b), and at 100°C/min (solid line), 300°C/min (dotted line), and at 500°C/min (dashed line) (Fig. 2c). The slightly thicker line in the graph reflects an exothermic event occurring at about 8°C and believed to be due to crystallization of amorphous calcipotriol.

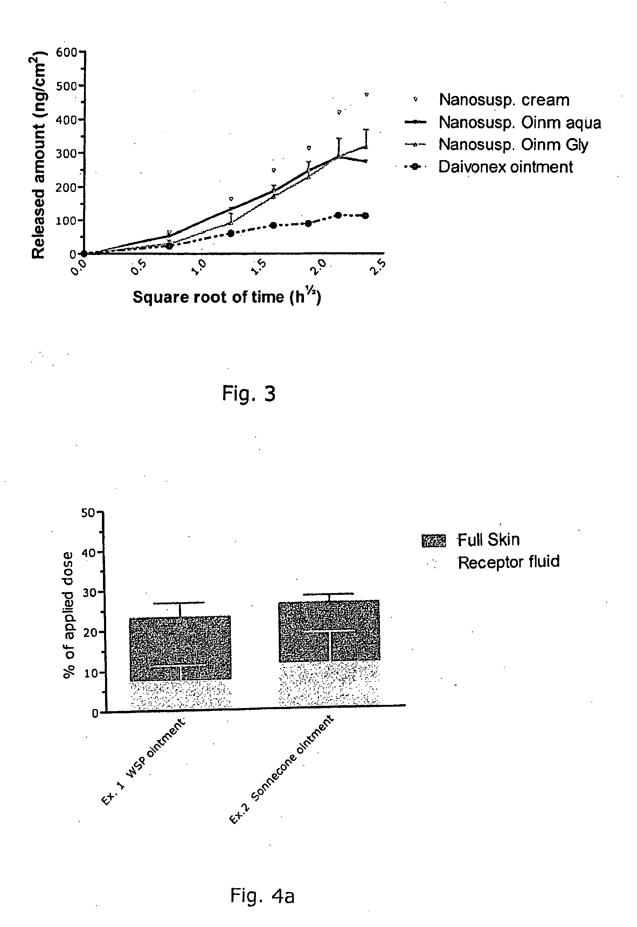

Fig. 3 is a graph showing the release rate of calcipotriol from the present nanosuspensions compared to the release rate from Daivonex^{®} ointment. It appears from the figure that the release rate is significantly higher from the nanosuspension formulations than from Daivonex^{®} ointment. "Nanosuspension cream" is the cream according to Example 3. "Nanosusp. oinm. aqua" corresponds to Composition A of Example 2 without glycerol, while "Nanosusp. oinm. gly" is Composition A of Example 2.
Fig. 4a is a graph showing the penetration into the skin and flux through the skin from two nanosuspension ointments, Composition A and C of Example 2. "WSP ointment" is Composition A, while "Sonnecone ointment" is Composition C.

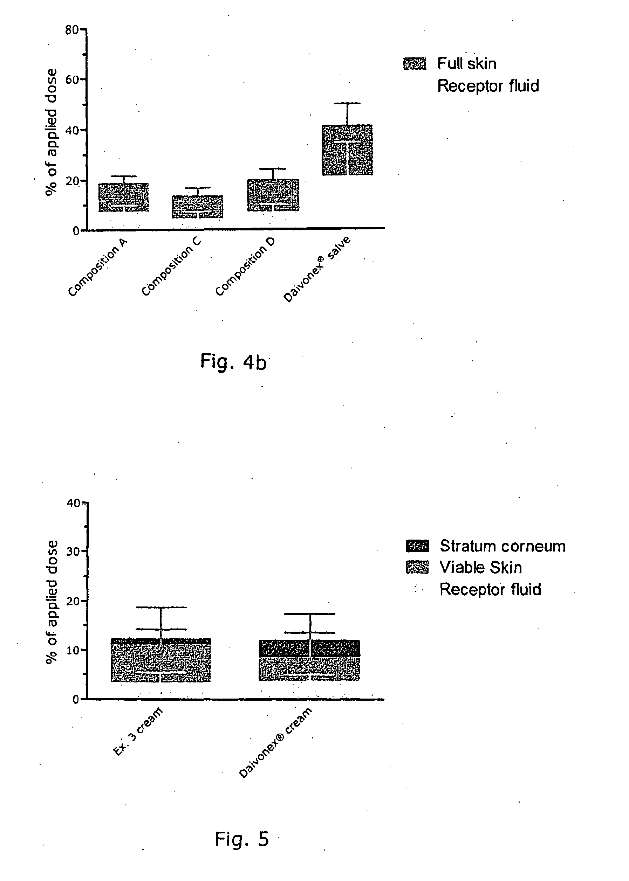

Fig. 4b is a graph showing the penetration into the skin and flux through the skin of calcipotriol from nanosuspension ointments, Composition A, C and D, of the invention compared to Daivonex^{®} ointment. It appears from the figure that the penetration into viable skin from the nanosuspension ointments is comparable to that from Daivonex^{®} ointment, while the flux is significantly lower, resulting in less systemic exposure to calcipotriol.
Fig. 5 is a graph showing the penetration into the skin and flux through the skin of calcipotriol from a nanosuspension cream of the invention compared to Daivonex^{®} cream. It appears from the figure that the penetration of calcipotriol from the nanosuspension cream into viable skin is significantly higher from the nanosuspension cream than from Daivonex^{®} cream.

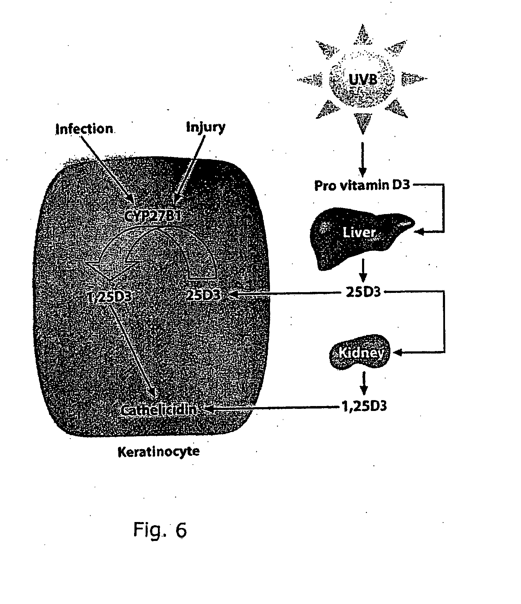

Fig. 6 is a schematic representation of the activation of the gene encoding cathelicidin by vitamin D₃ in human keratinocytes. The mechanism of cathelicidin gene activation is used in a biological assay using reconstructed human epidermis (human keratinocytes cultured so as to form the epidermal layers characteristic of human skin) on which calcipotriol-containing compositions of the invention are applied to activate cathelicidin as described in detail in Example 8 below.

### DETAILED DESCRIPTION OF THE INVENTION

### Definitions

In the present context, the term "nanocrystals" is intended to mean crystal particles of calcipotriol monohydrate, which are in the nanosize range, i.e. between 1 and 1000 nm in diameter. The nanocrystals favourably have a particle size distribution such that ≥ 90% of the nanocrystals have a particle size between 100 and 900 nm, in particular between 200 and 600 nm.

The term "nanosuspension" is intended to mean nanocrystals as defined above suspended in an aqueous phase.

The term "particle size distribution" is intended to mean the span between the smallest and largest calcipotriol monohydrate crystal as determined by dynamic light scattering (also known as photon correlation spectroscopy) using a Zetasizer Nano ZS or ZS90 according to the manufacturer's instructions (available from Malvern Instruments, UK). Dynamic light scattering determines the size of solid particles suspended in a liquid by illuminating the particles with a laser and analysing the intensity fluctuations in the scattered light resulting from the Brownian motions of the particles in the liquid. Intensity fluctuations are correlated to particle size in that larger particles move more slowly than smaller particles, i.e. the intensity fluctuation is slower.

The term "amorphous" is intended to mean a solid substance without an ordered arrangement of its molecules, i.e. the opposite of crystalline.

"Calcipotriol" is a vitamin D analogue of the formula

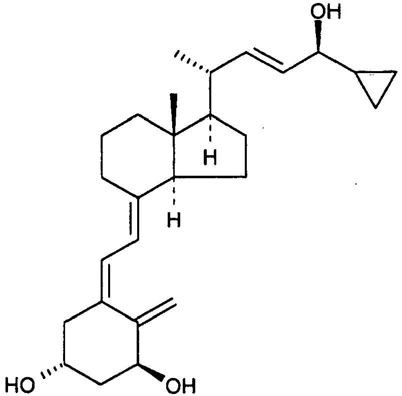

Calcipotriol has been found to exist in two crystalline forms, an anhydrate and a monohydrate. Calcipotriol monohydrate and its preparation are disclosed in WO 94/15912.

The term "chemical stability" or "chemically stable" is intended to mean that the calcipotriol monohydrate nanocrystals do not degrade significantly over time to 24-epi calcipotriol or other degradation products of calcipotriol in suspension or in the finished pharmaceutical product. In case of the latter, "chemical stability" indicates that no more than 10%, preferably no more than 6%, of the calcipotriol monohydrate degrades over the shelf-life of the product, typically 2 years, at room temperature. An approximation of chemical stability at room temperature is obtained by subjecting the nanocrystals or a composition containing them to accelerated stability studies at 40°C. If less than about 10% of the substance has degraded after 3 months at 40°C, this is usually taken to correspond to a shelf-life of 2 years at room temperature.

The term "physical stability" or "physically stable" is intended to mean that the calcopotriol monohydrate nanocrystals have essentially the identical crystal form to that of the reference calcipotriol monohydrate, which has not been subjected to nanosizing, as determined by Raman spectroscopy, i.e. it does not exhibit polymorphism as a result of nanosizing. Furthermore, "physical stability" indicates that the nanocrystals do not exhibit aggregation or crystal growth in the claimed suspensions or pharmaceutical compositions into which they are incorporated.

The term "substantially non-aqueous" is intended to mean that the content of free water (as opposed to crystal-bound water) freeze-dried or spray-dried calcipotriol monohydrate nanocrystals is less than about 2% by weight, preferably less than about 1% by weight, such as less than about 0.5% by weight, of the nanocrystals. Similarly, the content of free water in a "substantially anhydrous" ointment composition is less than about 3% by weight, preferably less than about 2% by weight, such as less than about 1% or 0.5% by weight, of the composition.

The term "solubilization capacity" is intended to, indicate the ability of a solvent or mixture of solvents to dissolve a given substance, expressed as the amount required to effect complete solubilization of the substance.

The term "skin penetration" is intended to mean the diffusion of the active ingredient into the different layers of the skin, i.e. the stratum corneum, epidermis and dermis.

The term "skin permeation" is intended to mean the flux of the active ingredient through the skin into the systemic circulation or, in case of *in vitro* studies such as those reported in Example 7 below, the receptor fluid of the Franz cell apparatus used in the experiment.

The term "biological activity" is intended to mean the activity of a vitamin D derivative or analogue when applied to skin in a composition of the invention. The biological activity of compositions is determined in an *in vitro* assay measuring the activation of a target gene encoding the biomarker cathelicidin in reconstructed human epidermis involving cultured human keratinocytes, as described in detail in Example 8 below.

### Preparation of calcipotriol monohydrate nanocrystals

In recent years the preparation of nanocrystals or nanosuspensions of therapeutically active ingredients has been increasingly investigated as a way of providing an improved dissolution rate of poorly soluble drugs. The larger surface area of nanocrystals ensures a higher dissolution velocity when the drug is administered. The technology has hitherto mostly been used in the formulation of active ingedients for oral or intravenous administration.

Several methods of making drug nanocrystals have been described in the literature. Broadly, the methods may be divided into two categories, i.e. milling and high-pressure homogenization.

US 5,145,684 discloses a method of preparing crystalline nanoparticles by ball milling for 4-5 days in the presence of surface modifiers such a polyvinyl pyrrolidone, polyvinyl alcohol, lecithin or other surfactant. Ball milling of calcipotriol monohydrate under these conditions is likely to result either directly in chemical degradation of the calcipotriol monohydrate or in the formation of large amounts of amorphous calcipotriol, which would not be favourable for a sufficient storage stability/shelf-life of a pharmaceutical composition containing it as amorphous material is relatively more vulnerable to chemical degradation than crystalline material.

CA 2375992 discloses a method of preparing drug particles with a particle size of less than 5µm, preferably less than 1µm, by high pressure homogenization in a piston-gap homogenizer in an anhydrous medium at a temperature below 20°C, in particular below 0°C. Nanosizing is carried out by subjecting micronized drug particles to 10-20 cycles of high pressure homogenization at 1500 bar. In the present method, we use an aqueous medium for the diminution of calcipotriol as using an anhydrous medium (liquid paraffin) did not result in any significant size reduction of the calcipotriol monohydrate crystals.

WO 2004/054549 discloses a topical formulation of spironolactone nanoparticles in a cream base comprising monoglycerides in water. The nanoparticles are prepared using piston gap high pressure homogenization.

High pressure homogenization at 1500 bar for several cycles has been found to be unsuitable for the diminution of calcipotriol monohydrate as this procedure leads to aggregation of the calcipotriol monohydrate crystals even in the presence of a suitable surfactant.

WO 2008/058755 discloses the preparation of nanocrystals of cosmetically active substance by pearl or ball milling followed by high pressure homogenization. The combination of the two methods is indicated to be advantageous over high pressure homogenization on its own as the latter requires many cycles of homogenization at high pressure (1500 bar). The combination method makes it possible to use only one cycle of homogenization at lower pressure to obtain nanosized particles.

In a favoured embodiment, the invention relates to a process for preparing calcipotriol monohydrate nanocrystals of a particle size distribution in the range of 200-600 nm as determined by dynamic light scattering, the process comprising the steps of
(a) diminuting crystalline calcipotriol monohydrate in an aqueous phase comprising non-ionic, polymeric surfactant in an amount in the range of from about 1% to about 5% by weight of said aqueous phase, resulting to the formation of microparticles with a particle size distribution in the range of about 5-20 µm and a mean particle size of about 10 µm;
(b) subjecting the suspension of step (a) to a first cycle of high pressure homogenization at a pressure of about 300-800 bar for a period of time sufficient to obtain about 15-40% of crystals of calcipotriol monohydrate with a particle size distribution in the range of 200-600 nm;
(c) subjecting the suspension of step (b) to a second cycle of high pressure homogenization at a pressure of about 800-1200 bar a period of time sufficient to obtain about 40-80% of crystals of calcipotriol monohydrate with a particle size distribution in the range of 200-600 nm;
(d) subjecting the suspension of step (c) to a third cycle of high pressure homogenization at a pressure of about 1200-1700 bar a period of time sufficient to obtain about 90% or more of crystals of calcipotriol monohydrate with a particle size distribution in the range of 200-600 nm; and
(e) optionally isolating the resulting nanocrystals of calcipotriol monohydrate from the aqueous phase.

In the final suspension (after step (d)) the amount of calcipotriol monohydrate nanocrystals with a particle size distribution in the range of 200-600 nm is preferably about 95% or more.

The present method differs from that disclosed in WO 2008/058755 by combining the initial diminution step (a) with three successive cycles of high pressure homogenization, each cycle being carried out at an increasing pressure. This is unlike the preferred procedure of WO 2008/058755 where ball milling of the active ingredient is followed by one cycle of high pressure homogenization at low pressure (such as 100 bar, cf Examples 8 and 9) resulting in the desired particle size reduction. Such a procedure has been found to be insufficient to provide a satisfactory particle size and particle size distribution of calcipotriol monohydrate crystals, i.e. only about 15-40% of the crystals would be within the desired particle size distribution.

Furthermore, it has been found unnecessary to carry out the present process with temperature control unlike the procedure disclosed in WO 2008/058755 where, for two out of three active ingredients, the temperature had to be maintained below 20°C and ideally between 0°C and 5°C. Not having to apply temperature control offers the advantage of a simplified procedure. It is surprising, however, that temperature control is not required in the present process as calcipotriol is sensitive to temperature increases and would be expected to be chemically degraded without temperature control of the diminution process.

### Embodiments of the Invention

The present suspension may contain a non-ionic, polymeric surfactant which is added to prevent aggregation of the nanocrystals of calcipotriol and/or to prevent crystal growth. The surfactant should preferably be one that does not cause any significant solubilization of the calcipotriol monohydrate nanocrystals, i.e. it should have a poor solubilization capacity, and may favourably be selected from the group consisting of poloxamer or polysorbate surfactants, and polyoxyethylene C₆₋₂₄ alkyl ethers. The poloxamer may be selected from the group consisting of poloxamer 124, poloxamer 188, poloxamer 237, poloxamer 338 and poloxamer 407. In particular, poloxamer 188 has been found to have a poor solubilization capacity with respect to solubilizing calcipotriol and is therefore the currently favoured surfactant for use in the present nanosuspension. When using a polysorbate as the surfactant, it may be selected from the group consisting of polysorbate 20, polysorbate 40, polysorbate 60, polysorbate 61, polysorbate 80 and polysorbate 81. The currently preferred polyoxyethylene C₆₋₂₄ alkyl ether is cetomacrogol 1000.

The amount of surfactant in the aqueous phase may be in the range of from about 0.01% to about 5% by weight of the suspension. It is generally preferred that the amount of surfactant in said aqueous phase is in the range of about 0.6-1.2% by weight of the suspension.

Dependent on the conditions applied during diminution and high pressure homogenization, the calcipotriol monohydrate nanocrystals present in the resulting suspension may have a mean particle size of 200-350 nm, 350-400 nm or 400-500 nm as determined by dynamic light scattering.

The nanosuspension may be freeze-dried or spray-dried to nanocrystals comprising surfactant on the surface. The freeze-dried or spray-dried nanocrystals may then be used for incorporation in non-aqueous compositions.

It has surprisingly been found that the present nanosizing procedures result in the generation of insignificant amounts only of amorphous calcipotriol. It is well known to the person skilled in the art that the presence of amorphous compound makes the material unstable due to the lack of molecular arrangement in a crystal lattice which exposes the material to chemical degradation or rearrangement of the crystal structure to a different polymorphic form (cf. Chow et al., J. Pharm. Sci. 97(8), 2008, pp. 2855-2877). As determined by differential scanning calorimetry using a Perkin-Elmer DSC 8500 instrument in accordance with the manufacturer's instructions, it was not possible to detect significant amounts of amorphous calcipotriol to the limit of detection of the instrument (about 5%), cf. Fig. 2b.

In the present process, crystals of calcipotriol are initially subjected to milling, or premilling, in an aqueous phase using balls or beads of a diameter in the range of 1-4 mm, such as 2-3 mm. The balls or beads may be composed of glass or a similarly hard material such as zirconium oxide. Milling may suitably be performed for 2-5 hours, such as 3 hours, at about 500-4000 rpm, such as about 1000-3000 rpm, e.g. about 2000 rpm.

The surfactant used for milling may suitably be a non-ionic, polymeric surfactant which is added to the aqueous phase in an amount in the range of from about 1.5 to about 3% by weight of the suspension, in particular about 2% by weight of the suspension. The surfactant may preferably be selected from the group of poloxamer or polysorbate surfactants as described above. The suspension is subsequently used directly in the high pressure homogenization steps, and a particularly favourable result with respect to particle size distribution subsequent to high pressure homogenization has been obtained using poloxamer 188. It should be noted that because of the present manufacturing process where the milling equipment used in step (a) is rinsed with water, and the high pressure homogenization equipment is rinsed with water after step (d), the concentration of surfactant in the final suspension is in the range of about 0.6-1.2% by weight of the suspension.

The high pressure homogenization steps (b)-(d) are carried out using a piston gap homogenizer, e.g. Emulsiflex C3 (available from Avestin) in accordance with the manufacturer's instructions.

For the nanosizing of calcipotriol monohydrate it has been found favourable that the first cycle of high pressure homogenization of step (b) is carried out at a pressure of about 500-650 bar. The time required to obtain 15-40% calcipotriol monohydrate nanocrystals with the desired particle size distribution is in the range of 7-15 minutes, e.g. 8-12 minutes, such as about 10 minutes.

The second cycle of high pressure homogenization of step (c) may suitably carried out at a pressure of about 1000-1100 bar. The time required to obtain 40-80% calcipotriol monohydrate nanocrystals with the desired particle size distribution is in the range of 7-15 minutes, e.g. 8-12 minutes, such as about 10 minutes,

The third cycle of high pressure homogenization of step (d) may suitably be carried out at a pressure of about 1400-1500 bar. The time required to obtain 90% or more calcipotriol monohydrate nanocrystals with the desired particle size distribution is in the range of 7-15 minutes, e.g. 8-12 minutes, such as about 10 minutes.

If the calcipotriol monohydrate nanocrystals are intended for inclusion in a non-aqueous formulation, they may suitable be subjected to freeze-drying or spray-drying (to a water content (free water) of less than about 2% by weight, such as less than about 1% or less than about 0.5% by weight, of the nanocrystals.

The suspension comprising the nanocrystals, may be included in a pharmaceutical composition comprising a pharmaceutically acceptable carrier which is compatible with the active ingredient.

When mixed with pharmaceutically acceptable excipients to provide a composition as described below, the amount of the non-ionic polymeric surfactant is preferably in the range of about 0.03-0.06% by weight of the composition.

In one embodiment, the present compositon is an ointment. According to the current FDA classification, an ointment is a semisolid dosage from which may contain water and volatile substances in an amount of up to 20% by weight and which contains more than 50% by weight of hydrocarbons, waxes or polyols in the vehicle. Thus, according to the invention, the ointment may be a water-in-oil composition in which case the nanosuspension may be added as such to the lipophilic components of the composition, such that the composition contains up to 10% by weight or, preferably, up to 5% by weight of the aqueous phase. Alternatively, the composition may be a non-aqueous ointment which contains less than about 2%, preferably less than 1%, of free water by weight of the composition.

The ointment carrier may suitably contain a paraffin selected from paraffins consisting of hydrocarbons with chain lengths from C₅₋₆₀ and mixtures thereof. A frequently used ointment carrier is petrolatum, or white soft paraffin, which is composed of hydrocarbons of different chain lengths, peaking at about C₄₀₋₄₄, or a mixture of petrolatum and liquid paraffin (consisting of hydrocarbons of different chain lengths peaking at C₂₈₋₄₀). While petrolatum provides occlusion of the treated skin surface, reducing transdermal loss of water and potentiating the therapeutic effect of the active ingredient in the composition, it tends to have a greasy and/or tacky feel which persists for quite some time after application, and it is not easily spreadable. It may therefore be preferred to employ paraffins consisting of hydrocarbons of a somewhat lower chain length, such as paraffins consisting of hydrocarbons with chain lengths peaking at C₁₄₋₁₆, C₁₈₋₂₂, C₂₀₋₂₂, C₂₀₋₂₆ or mixtures thereof. It has been found that such paraffins are more cosmetically acceptable in that they are less tacky and/or greasy on application and more easily spreadable. They are therefore expected to result in improved patient compliance. Suitable paraffins of this type are manufactured by Sonneborn and marketed under the trade name Sonnecone, e.g. Sonnecone CM, Sonnecone DM1, Sonnecone DM2 and Sonnecone HV. These paraffins are further disclosed and characterized in WO 2008/141078. (The hydrocarbon composition of the paraffins has been determined by gas chromatography.)

To impart a desired viscosity to the present composition, it may suitably include a lipophilic viscosity-increasing ingredient such as a wax. The wax may be a mineral wax composed of a mixture of high molecular weight hydrocarbons, e.g. saturated C₃₅₋₇₀ alkanes, such as microcrystalline wax. Alternatively, the wax may be a vegetable or animal wax, e.g. esters of C₁₄₋₃₂ fatty acids and C₁₄₋₃₂ fatty alcohols, such as beeswax. The amount of viscosity-increasing ingredient may vary according to the viscosifying power of the ingredient, but may typically be in the range of about 1-20% by weight of the composition. When the viscosity-increasing ingredient is microcrystalline wax it is typically present in an amount in the range of about 5-15% by weight, e.g. about 10% by weight, of the composition.

To maintain good physical stability of the composition, in particular to avoid separation of the aqueous and lipid phases therein, it may be advantageous to include a water-in-oil emulsifier with an HLB value of 3-8. Examples of such emulsifiers are polyoxyethylene C₈₋₂₂ alkyl ethers, e.g. polyoxyethylene stearyl ether, polyoxyethylene cetyl ether, polyoxyethylene oleyl ether or polyoxyethylene lauryl ether. The amount of emulsifier is typically in the range of 2-10 % w/w of the composition.

The composition may additionally comprise an emollient which may act to soften the thickened epidermis of the psoriatic plaques. A suitable emollient for inclusion in the present composition may be a silicone wax or a volatile silicone oil as the presence of silicone has additionally been found to aid penetration of calcipotriol into the skin. Compositions including a silicone have also been found to result in less skin irritation. Suitable silicone oils for inclusion in the present composition may be selected from cyclomethicone, dimethicone. The amount of silicone oil included in the present composition is typically in the range of from about 1 to about 10% by weight, e.g. about 5% by weight, of the composition.

In Daivonex^{®} ointment, the presence of propylene glycol is believed to be a major contributor to the skin irritation experienced by many patients. However, it has been found that calcipotriol may in itself be mildly irritative in some patients (A. Fullerton and J. Serup, Br. J. Dermatol. 137, 1997, pp. 234-240 and A. Fullerton et al., Br. J. Dermatol. 138, 1998, pp. 259-265). It may therefore be advantageous to include an anti-irritant compound in the present composition, such as glycerol, sorbitol, sucrose, saccharin, menthol, eucalyptol or nicotinamide. Glycerol has been described as a substance that is capable of protecting the skin against irritative substances (J. Bettinger et al., Dermatology 197, 1998, pp. 18-24) and has been found by us to reduce the release of IL-1a in a dose-dependent manner: thus, it has been found that the presence of 15% by weight of glycerol in a calcipotriol ointment results in a significantly lower level of release of IL-1a than does the inclusion of 10% by weight of glycerol which, in turn, results in a significantly lower level of IL-1a release than does the inclusion of 5% by weight of glycerol.

However, in addition to the anti-irritative effect, it has surprisingly been found that glycerol is capable of potentiating the biological activity of calcipotriol in that the expression of cathelicidin (in the assay described in Example 4 below) has been found to be increased with a low amount of glycerol in the composition (i.e. more cathelicidin is expressed when the amount of glycerol is 5% by weight than when the amount of glycerol is 10% or 15%, respectively): this implies that with respect to inclusion of glycerol a balance has to be struck between a favourable anti-irritative effect and a favourable potentiating effect. We have found that the inclusion of about 5-10% by weight of glycerol in the present composition results in a significant anti-irritative effect as well as a significant potentiation of the biological activity of calcipotriol.

Calcipotriol is known to be a substance which is extremely sensitive to acid conditions (pH below about 7.0 in aqueous compositions or acidic reacting substances in non-aqueous compositions) which contribute to the rapid degradation of calcipotriol. To ensure an adequate chemical stability of the substance throughout the shelf-life of the composition, it may be advisable to include a compound capable of neutralizing acidic impurities which may be present in one or more of the excipients of the composition and which are detrimental to the chemical stability of calcipotriol. If the composition is aqueous, the acid neutralizing compound may be selected from a buffer such as a phosphate buffer which may be included in an amount of 0.025-0.065% by weight of the composition. If, on the other hand, the composition is non-aqueous, the acid neutralizing compound may advantageously be an amine such as triethanolamine, trometamol, monoethanolamine or diethanolamine, included in the composition in an amount of 0.1-2% by weight of the composition.

In another embodiment, the present composition is a cream which may comprise similar components to the ointment, but which is typically an oil-in-water-emulsion containing a substantial amount of water.

In a specific embodiment, the present composition comprises
0.003-0.008% w/w of calcipotriol mohohydrate
2-8% w/w of polyoxyethylene stearyl ether
2-10% w/w of water
0.001-0.005% w/w of poloxamer 188
70-90% w/w of paraffin carrier

The present composition may also comprise other components commonly used in dermal formulations, e.g. antioxidants (e.g. alpha-tocopherol), preservatives, sodium edetate, pigments, skin soothing agents, skin healing agents and skin conditioning agents such as urea, allantoin or bisabolol, cf. CTFA Cosmetic Ingredients Handbook, 2nd Ed., 1992.

The composition of the invention may be used in the treatment of psoriasis, sebopsoriasis, pustulosis palmoplantaris, dermatitis, ichtyosis, rosacea and acne and related skin diseases by topically administering an effective amount of a composition according to the invention to a patient in need of such treatment. Said method preferably comprises topical administration once or twice a day of a therapeutically sufficient dosage of said composition. To that end, the composition according to the invention preferably contains about 0.001-0.5 mg/g, preferably about 0.002-0.25 mg/g, in particular 0.005-0.05 mg/g, of calcipotriol monohydrate nanocrystals. It is envisaged that the present composition may advantageously been used for maintenance treatment of these dermal diseases, i.e. continued treatment after the diseappearance of visible symptoms to delay the recurrence of symptoms.

To provide a more effective treatment of psoriasis and other dermal conditions in the acute phase, it may be desirable to include one or more additional therapeutically active ingredients in the composition. Examples of such additional active ingredients include, but are not limited to, anti-inflammatory drugs such as corticosteroids, such as betamethasone and esters thereof, e.g. the valerate or dipropionate ester, clobetasol or esters thereof, such as the propionate, hydrocortisone or esters thereof, such as the acetate; non-steroidal anti-inflammatory drugs such as naproxen, indomethacin, diclofenac, ibuprofen, dexibuprofen, ketoprofen, flurbiprofen, piroxicam, lornoxicam or nabumeton, phosphodiesterase 4 inhibitors (e.g. the compounds disclosed in WO 2008/077404, WO 2008/104175, WO 2008/128538 or WO 2010/069322) or p38 MAP kinase inhibitors (e.g. the compounds disclosed in WO 2005/009940 or WO 2006/063585).

The invention is further illustrated by the following examples which are not in any way intended to limit the scope of the invention as claimed.

### EXAMPLES

### Example 1

### Preparation of calcipotriol monohydrate nanocrystals

4 g of poloxamer 188 was dissolved in 196 ml of laboratory water with stirring, and the pH was adjusted to 8.5 by adding an appropriate amount of NaOH.

3.5 g of 2 mm glass balls was weighed into two vials provided with a screw cap. 0.035 g of calcipotriol monohydrate was added to each vial, after which 1.05 g of the 2% poloxamer 188 solution was added to each vial. The calcipotriol monohydrate was milled by shaking on an orbital shaker (VXR Basic IKA Vibrax) at 2000 rpm.

After milling, the vials and glass balls used for milling were rinsed with 24.0 g of laboratory water, pH 8.5, and the calcipotriol monohydrate suspension was poured into a Blue Cap bottle. The suspension was transferred to an Emulsiflex C3 (Avestin) high pressure homogenizer, and the Blue Cap bottle was rinsed with 4.9 g of laboratory water, pH 8.5. High pressure homogenization was carried out at 500 bar for 10 minutes, at 1000 bar for 10 minutes and at 1400 bar for 10 minutes. After high pressure homogenization, the cylinder of the Emulsiflex apparatus was rinsed with 4.9 g of laboratory water, pH 8.5, after which the particle size distribution was determined by dynamic light scattering using a Zetasizer Nano ZS90 to be in the range of 200-600 nm and the mean particle size to be in the range of 350-400 nm.

The resulting nanocrystals were determined to be calcipotriol monohydrate by Raman spectroscopy, comparing the Raman spectrum of the nanocrystals with that of calcipotriol monohydrate that had not been subjected to nanosizing.

The amount of amorphous calcipotriol generated in this method was determined on two batches of calcipotriol nanocrystals prepared by the method using DSC analysis at a heating rate of 100°C, 300°C and 500°C/min. under a N₂ atmosphere. The instrument used for the analysis was a Perkin Elmer DSC 8500.

The results are shown in Fig. 2b and 2c showing an exothermic event with an onset at about 8°C. It is considered highly likely that the exothermic event is due to crystallization of amorphous calcipotriol. It appears that the amount of heat emitted during the crystallization process is very small, in fact very close to the limit of detection. Since the amount of heat emitted during the crystallization process is proportional to the amount of amorphous compound present in the sample, we concluded that only insignificant amounts of amorphous calcipotriol were present in the two batches.

### Example 2

### Ointments containing calcipotriol monohydrate nanocrystals

Ointments of the composition shown in Table 1 below were prepared by mixing the ingredients of the lipid phase (hydrocarbons + polyoxyethylene-2-stearyl ether + a-tocopherol) with heating to 80-85°C and slow agitation. The aqueous phase was prepared by dissolving disodium edetate and disodium phosphate dihydrate in the appropriate amount of aqueous calcipotriol monohydrate nanosuspension (prepared as described in Example 1) adjusted to contain 50 µg/g calcipotriol monohydrate. Glycerol was added to the suspension with mixing and heating to 35-40°C and the pH of the mixture was adjusted to 8.5 with 1N HCl or NaOH, as appropriate.

The aqueous phase was added to the lipid phase with whisking for 30 min. after which the resulting ointment was cooled slowly to below 32°C and filled into aluminium tubes and stored at room temperature.

**Table 1**

| Ingredient (mg/g) | Comp.A | Comp.B | Comp.C | Comp.D | Comp.E | Comp.F |
|---|---|---|---|---|---|---|
| Calcipotriol monohydrate nanocrystals | 0.05 | 0.05 | 0.05 | 0.05 | 0.05 | 0.05 |
| Disodium phosphate dehydrate | 0.26 | 0.26 | 0.26 | 0.26 | 0.26 | 0.26 |
| Paraffin, liquid | 50 | 50 | 50 | 50 | 50 | 50 |
| Polyoxyethylene-2-stearyl ether | 50 | 50 | 50 | 50 | 50 | 50 |
| Disodium edetate | 0.065 | 0.065 | 0.065 | 0.065 | 0.065 | 0.065 |
| all-rac-a-tocopherol | 0.02 | 0.02 | 0.02 | 0.02 | 0.02 | 0.02 |
| Glycerol | 100 | - | 100 | 100 | 100 | 100 |
| Water, purified | 50 | 150 | 50 | 50 | 50 | 50 |
| Poloxamer 188 | 0.03 | 0.03 | 0.03 | 0.03 | - | - |
| Poloxamer 407 | - | - | - | - | 0.03 | - |
| Polysorbate 80 | - | - | - | - | - | 0.03 |
| Paraffin, white soft | 749.6 | - | - | 699.6 | 749.6 | 749.6 |
| Cyclomethicone | - | - | - | 50 | - | - |
| Petrolatum Jelly White (Sonnecone DM1) | - | 649.6 | 649.6 | - | - | - |
| Microcrystalline wax (Multiwax 180 MH) | - | 100 | 100 | - | - | - |

The compositions were tested for chemical stability for 3 months at 40°C/75% RH. The results show a satisfactory stability of calcipotriol under the test conditions.

### Example 3

### Cream containing calcipotriol monohydrate nanocrystals

A cream of the composition indicated below in Table 2 was prepared by melting cetomacrogol 1000, cetostearylalcohol, liquid paraffin and white soft paraffin at 80°C. The aqueous phase was prepared by dissolving disodium phosphate dihydrate and chloroallylhexaminium chloride in purified water at 80°C. Glycerol was added to the solution with mixing, and the pH of the mixture was adjusted to 8.5 with 1N HCl or NaOH, as appropriate.

The aqueous phase was mixed with the lipid phase with homogenization and cooled to 55°C. The remaining water was added with vigorous stirring, and the resulting cream was cooled to 25°C while stirring at slow speed.

An appropriate amount of the calcipotriol monohydrate nanosuspension (prepared as described in Example 1) adjusted to contain 50 µg/g calcipotriol monohydrate was added to the cream with mixing for 30 minutes at <30°C. The resulting cream was filled into tubes and stored until further use.

**Table 2**

| Ingredient (mq/q) | Comp. G | Comp. H | Comp. I |
|---|---|---|---|
| Calcipotriol monohydrate nanocrystals | 0.05 | 0.05 | 0.05 |
| Cetomacrogol 1000 | 30 | 30 | 30 |
| Cetostearylalcohol | 60 | 60 | 60 |
| Chloroallylhexaminium chloride | 0.5 | 0.5 | 0.5 |
| Glycerol | 30 | 30 | 30 |
| Disodium phosphate dihydrate | 2 | 2 | 2 |
| Poloxamer 188 | 0.03 | - | - |
| Poloxamer 407 | - | 0.03 | - |
| Polysorbate 80 | - | - | 0.03 |
| Paraffin, liquid | 50 | 50 | 50 |
| Paraffin, white soft | 170 | 170 | 170 |
| Water, purified | ad 1 g | ad 1 g | ad 1 g |

The cream compositions were tested for chemical stability for 3 months at 40°C/75% RH. The results show a satisfactory stability of calcipotriol under the test conditions.

### Example 4

### Non-aqueous ointment containing calcipotriol monohydrate nanocrystals

The calcipotriol monohydrate nanosuspension prepared as described in Example 1 was subjected to freeze-drying overnight. The freeze-dried, substantially anhydrous nanocrystals were used to prepare an ointment by dispersing the nanocrystals in liquid paraffin and adding white soft paraffin to the dispersion.

The composition of the non-aqueous ointment appears from Table 3 below.

**Table 3**

| Ingredient | Content |
|---|---|
| Calcipotriol monohydrate nanocrystals | 50 µg |
| Paraffin, liquid | 50 mg |
| Paraffin, white soft | 750 mg |
| Poloxamer 188 | 0.05 mg |

The non-aqueous ointment was tested for stability for 3 months at 40°C/75% RH. The results show a satisfactory stability of calcipotriol under the test conditions.

### Example 6

### Release from nanosuspension compositions compared to Daivonex^{®} ointment

*In vitro* release of calcipotriol from the compositions described in Examples 1 and 2 was determined in diffusion cells of Plexiglass using a Spectra/Porv 6 membrane to separate the receptor and donor chambers (n=6 cells per batch). The release of calcipotriol into a recipient phase consisting of 0.04M isotonic phosphate buffer, pH 7.4, and isopropanol (70:30 v/v) was determined. The samples were analysed by HPLC/UV.

The results which appear from Fig. 3 below, show that the release rate of calcipotriol from the present nanosuspension ointments and cream is significantly higher than the release rate from Daivonex^{®} ointment.

The results shown in Fig. 3 show that the release rate is significantly higher from the nanosuspension formulations than from Daivonex^{®} ointment.

### Example 7

### In vitro skin penetration study

To investigate the skin penetration and permeation of calcipotriol from compositions of the invention, a skin diffusion experiment was conducted. Full thickness skin from pig ears was used in the study. The ears were kept frozen at -18°C before use. On the day prior to the experiment the ears were placed in a refrigerator (5±3°C) for slow defrosting. On the day of the experiment, the hairs were removed using a veterinary hair trimmer. The skin was cleaned for subcutaneous fat using a scalpel and two pieces of skin were cut from each ear and mounted on Franz diffusion cells in a balanced order.

Static Franz-type diffusion cells with an available diffusion area of 3.14 cm² and receptor volumes ranging from 8.6 to 11.1 ml were used in substantially the manner described by T.J. Franz, "The finite dose technique as a valid in vitro model for the study of percutaneous absorption in man", in Current Problems in Dermatology, 1978, J.W.H. Mall (Ed.), Karger, Basel, pp. 58-68. The specific volume was measured and registered for each cell. A magnetic bar was placed in the receptor compartment of each cell. After mounting the skin, physiological saline (35°C) was filled into each receptor chamber for hydration of the skin. The cells were placed in a thermally controlled water bath which was placed on a magnetic stirrer set at 400 rpm. The circulating water in the water baths was kept at 35±1C resulting in a temperature of about 32°C on the skin surface. After one hour the saline was replaced by receptor medium, 0.04 M isotonic phosphate buffer, pH 7.4 (35°C), containing 4% bovine serum albumin. Sink conditions were maintained at all times during the period of the study, i.e. the concentration of the active compounds in the receptor medium was below 10% of the solubility of the compounds in the medium.

The *in vitro* skin permeation of each test composition was tested in 6 replicates (i.e. n=6). Each test composition was applied to the skin membrane at 0 hours in an intended dose of 4 mg/cm². A glass spatula was used for the application, and the residual amount of the composition was determined so as to give the amount of the composition actually applied on the skin.

The skin penetration experiment was allowed to proceed for 21 hours. Samples were then collected from the following compartments:
The stratum corneum was collected by tape stripping 10 times using D-Squame^{®} tape (diameter 22 mm, CuDerm Corp., Dallas, Texas, USA). Each tape strip is applied to the test area using a standard pressure for 5 seconds and removed from the test area in one gentle, continuous move. For each repeated strop, the direction of tearing off was varied. The viable epidermis and dermis was then sampled from the skin in a similar fashion.

Samples (1 ml) of the receptor fluid remaining in the diffusion cell were collected and analysed.

The concentration of calcipotriol in the samples was determined by LC mass spectrometry.

The results appear from Fig. 4a which shows the penetration into viable skin from Composition A and C using two different paraffin carriers, and Fig. 4b which shows that the penetration into viable skin from the nanosuspension ointments is comparable to that from Daivonex^{®} ointment, while the flux is significantly lower, resulting in less systemic exposure to calcipotriol.

Further results appear from Fig. 5 which is a graph showing that the penetration of calcipotriol from Composition G into viable skin is significantly higher from the nanosuspension cream than from Daivonex^{®} cream.

### Example 8

### Biological activity of the compositions

As shown in figure 6 below, cathelicidin is an antimicrobial peptide expressed in human keratinocytes. The expression of cathelicidin is strongly induced on infection of the skin or disruption of the skin barrier. In psoriasis, the level of cathelicidin is increased in lesional skin of psoriasis patients. It has been found that the expression of the gene encoding cathelicidin may be induced by vitamin D₃ or vitamin D analogues such as calcipotriol (cf. TT Wang et al, J. Immunol. 173(5), 2004, pp. 2909-2912; J Schauber et al., Immunology 118(4), 2006, pp. 509-519; Schauber and Gallo, J. Allergy Clin Immunol 122, 2008, pp. 261-266; M. Peric et al., PloS One 4(7), 22 July 2009, e6340) through binding to the vitamin D receptor. This finding has been utilized to develop an assay in which the uptake and biological activity of calcipotriol in human keratinocytes from the tested compositions has been determined by measuring the level of induction of the gene encoding cathelicidin.

In the assay, a calcipotriol monohydrate nanocrystal cream prepared as described in Example 3 above (Composition G) was applied topically in triplicate on reconstructed human epidermis consisting of normal human keratinocytes cultured for 12 days on 0.5 cm² polycarbonate filters (available from SkinEthic^{®} Laboratories, Nice, France) in an amount of 10 µl. The tissue was treated for one or two days in the presence of the cytokines IL-17A (20 ng/ml), IL-22 (20 ng/ml) and TNF-α (5 ng/ml) followed by separation of the epidermis from the polycarbonate filter and snap-frozen in liquid nitrogen. RNA was extracted from the cells and cDNA synthesized by conventional procedures. Quantitative real-time PCR (qPCR) was then performed using the following assays from Applied Biosystems: CAMP Hs0018038_m1 and GAPDH Hs99999905_m1. The expression levels of cathelicidin were normalized to GAPDH and a relative quantification was made by comparison with Daivonex^{®} ointment and cream.

The results appear from Table 4 below.

**Table 4**

| Composition | Activation¹ Day 1 | Activation¹ Day 2 |
|---|---|---|
| Daivonex^{®} ointment | 2.3 | 4.1 |
| Daivonex^{®} cream | 1.0 | 2.2 |
| Composition G | 2.5 | 4.7 |

| | | |
|---|---|---|
| ¹ fold activation relative to Daivonex^{®} cream, day 1 | | |

The results presented in Table 4 show that application of Composition G resulted in activation of the target gene similar to that obtained with Daivonex^{®} ointment while the activation of the target gene was about twice that of Daivonex^{®} cream. Thus, the results indicate an efficacy which is as good as that obtained for Daivonex^{®} ointment obtained with a formulation which does not contain propylene glycol and which has more favourable cosmetic properties.

Compositions A, C and D prepared as described in Example 2 above were applied topically in triplicate on reconstructed human epidermis consisting of normal human keratinocytes cultured for 12 days on 0.5 cm² polycarbonate filters (available from SkinEthic^{®} Laboratories, Nice, France) in an amount of 10 µl. The tissue was treated for two days followed by separation of the epidermis from the polycarbonate filter and snap-frozen in liquid nitrogen. RNA was extracted from the cells and cDNA synthesized by conventional procedures. qPCR was then performed using the following assays from Applied Biosystems: CAMP Hs0018038_m1 and GAPDH Hs99999905_m1. The expression levels of cathelicidin were normalized to GAPDH and a relative quantification was made by comparison with Daivonex^{®} ointment.

The results appear from Table 5 below.

**Table 5**

| Composition | Fold activation¹ |
|---|---|
| Daivonex^{®} ointment | 1.0 |
| composition A | 2.6/2.1 |
| composition C | 1.3/4.1 |
| composition D | 4.1/6.8 |

| | |
|---|---|
| ¹ relative to Daivonex^{®} ointment | |

The results presented in Table 5 show that the compositions of the invention result in higher activation of the target gene, i.e. they have a higher biological activity than the marketed ointment.

### Example 5

### Local tolerance study in minipigs

The local tolerability of compositions A, C and D of Example 2 was assessed when administered daily by dermal application to minipigs for 4 weeks. Daivonex^{®} ointment was used for comparison. Each day the animals were exposed to the test items for 8 hours.

The study was conducted in 10 female Göttingen SPF minipigs. Each animal had 6 application sites and received a volume of 250 mg test formulation per application site. Clinical signs were recorded daily and skin reactions at the application sites were scored once daily prior to start of dosing and, furthermore, on the day of necropsy in relation to erythema and oedema. Food consumption was recorded daily and the body weight weekly. At the end of the treatment period a gross necropsy was performed on all animals and skin samples were collected from histopathological examination.

The results show that no adverse treatment-related clinical signs were observed during the study though grade 1-2 skin reactions (erythema) were observed. Except for composition A, the erythemas were less pronounced than those observed for Daivonex^{®} ointment. The results imply that compositions of the invention may be better tolerated in human patients than Daivonex^{®} ointment.

## Claims

1. A suspension of calcipotriol monohydrate in the form of nanocrystals of a particle size distribution in the range of 200-600 nm as determined by dynamic light scattering, the suspension further comprising an aqueous phase including a non-ionic, polymeric surfactant in an amount sufficient to prevent formation of aggregates and/or crystal growth of the calcipotriol monohydrate nanocrystals.

2. The suspension according to claim 1, wherein the surfactant is selected from the group consisting of poloxamer or polysorbate surfactants, and polyoxyethylene C₆₋₂₄ alkyl ether.

3. The suspension according to claim 2, wherein the poloxamer is selected from the group consisting of poloxamer 124, poloxamer 188, poloxamer 237, poloxamer 338 and poloxamer 407.

4. The suspension according to claim 3, wherein the surfactant is poloxamer 188.

5. The suspension according to claim 2, wherein the polysorbate is selected from the group consisting of polysorbate 20, polysorbate 40, polysorbate 60, polysorbate 61, polysorbate 80 and polysorbate 81.

6. The suspension according to claim 2, wherein the polyoxyethylene C₆₋₂₄ alkyl ether is cetomacrogol 1000.

7. The suspension according to any of of claims 2-6, wherein the amount of surfactant in said aqueous phase is in the range of from about 0.6% to about 1.2% by weight of the suspension.

8. The suspension according to claim 1, wherein the calcipotriol monohydrate nanocrystals have a mean particle size of 200-350 nm, 350-400 nm or 400-500 nm as determined by dynamic light scattering.

9. A process for preparing calcipotriol monohydrate nanocrystals of a particle size distribution in the range of 200-600 nm as determined by dynamic light scattering, the process comprising the steps of
(a) diminuting crystalline calcipotriol monohydrate in an aqueous phase comprising non-ionic, polymeric surfactant in an amount in the range of from about 1% to about 5% by weight of said aqueous phase, resulting to the formation of microparticles with a particle size distribution in the range of about 5-20 µm and a mean particle size of about 10 µm;
(b) subjecting the suspension of step (a) to a first cycle of high pressure homogenization at a pressure of about 300-800 bar for a period of time sufficient to obtain about 15-40% of crystals of calcipotriol monohydrate with a particle size distribution in the range of 200-600 nm;
(c) subjecting the suspension of step (b) to a second cycle of high pressure homogenization at a pressure of about 800-1200 bar a period of time sufficient to obtain about 40-80% of crystals of calcipotriol monohydrate with a particle size distribution in the range of 200-600 nm;
(d) subjecting the suspension of step (c) to a third cycle of high pressure homogenization at a pressure of about 1200-1700 bar a period of time sufficient to obtain about 90% or more of crystals of calcipotriol monohydrate with a particle size distribution in the range of 200-600 nm; and
(e) optionally isolating the resulting nanocrystals of calcipotriol monohydrate from the aqueous phase.

10. The process of claim 9, wherein the diminution of step (a) is carried out by wet ball milling using balls or beads of a diameter in the range of 1-4 mm, such as 1.5-2.5 mm or 2-3 mm.

11. The process of claim 9, wherein the surfactant used in step (a) is selected from the group consisting of poloxamer or polysorbate surfactants, and polyoxyethylene C₆₋₂₄ alkyl ether.

12. The process of claim 11, wherein the poloxamer is selected from the group consisting of poloxamer 124, poloxamer 188, poloxamer 237, poloxamer 338 and poloxamer 407.

13. The process of claim 12, wherein the surfactant is poloxamer 188.

14. The process of claim 11, wherein the polysorbate is selected from the group consisting of polysorbate 20, polysorbate 40, polysorbate 60, polysorbate 61, polysorbate 80 and polysorbate 81.

15. The process of claim 11, wherein the polyoxyethylene C₆₋₂₄ alkyl ether is cetomacrogol 1000.

16. The process of any one of claims 9-15, wherein the surfactant is added in step (a) in an amount in the range of from about 1.5 to about 3% by weight of the suspension, in particular about 2% by weight of the suspension.

17. The process of claim 9, wherein the first cycle of high pressure homogenization of step (b) is carried out at a pressure of about 500-650 bar.

18. The process of claim 9, wherein the second cycle of high pressure homogenization of step (c) is carried out at a pressure of about 1000-1100 bar.

19. The process of claim 9, wherein the third cycle of high pressure homogenization of step (d) is carried out at a pressure of about 1400-1500 bar.

20. The process of any one of claims 9-19, wherein the high pressure homogenization steps (b)-(d) are carried out using a piston gap homogenizer.

21. The process of any one of claims 9-20 further comprising freeze-drying or spray-drying the calcipotriol monohydrate nanocrystals.

22. A pharmaceutical composition comprising the suspension of calcipotriol monohydrate nanocrystals of any one of claims 1-8 and a pharmaceutically acceptable carrier.

23. The composition according to claim 22, wherein the amount of non-ionic polymeric surfactant is in the range of about 0.03-0.06% by weight of the composition.

24. A composition according to claim 22, wherein the carrier comprises at least one paraffin selected from paraffins consisting of hydrocarbons with chain lengths from C₅₋₆₀, the chain lengths peaking at C₁₄₋₁₆, C₁₈₋₂₂, C₂₀₋₂₂, C₂₀₋₂₆, C₂₈₋₄₀ and C₄₀₋₄₄, or mixtures thereof.

25. A composition acording to any one of claims 22-24, further comprising a water-in-oil emulsifier selected from polyoxyethylene C₈₋₂₂ alkyl ethers, e.g. polyoxyethylene stearyl ether, polyoxyethylene cetyl ether or polyoxyethylene lauryl ether.

26. A composition according to any one of claims 22-25, further comprising a lipophilic viscosity-increasing ingredient.

27. A composition according to claim 26, wherein the viscosity-increasing ingredient is a wax.

28. A composition according to any one of claims 22-27, further comprising a silicone wax or a volatile silicone oil.

29. A composition according to claim 28, wherein the volatile, silicone oil is cyclomethicone or dimethicone.

30. A composition according to any one of claims 22-29, further comprising an anti-irritant compound.

31. A composition according to claim 30, wherein the anti-irritant compound is glycerol, sorbitol, sucrose, saccharin, nicotinamide, menthol or eucalyptol.

32. A composition according to any one of claims 22-31, further comprising a compound capable of neutralizing acidic impurities detrimental to the chemical stability of calcipotriol monohydrate in the composition.

33. A composition according to claim 32, wherein said compound is an amine such as triethanol amine, trometamol, monoethanolamine or dimethanolamine.

34. A composition according to any one of claims 22-33 which is an ointment.

35. A composition according to claim 34 which is a substantially anhydrous ointment.

36. A composition according to any one of claims 22-33 which is a cream.

37. A composition according to any one of claims 22-36 comprising about 0.001-0.5 mg/g, preferably about 0.002-0.25 mg/g, in particular 0.005-0.05 mg/g, of calcipotriol monohydrate nanocrystals.

38. A composition according to any one of claims 22-37, further comprising one or more additional therapeutically active ingredients.

39. A composition according to claim 38, wherein such additional active ingredients are selected from the group consisting of anti-inflammatory drugs such as corticosteroids, such as betamethasone and esters thereof, e.g. the valerate or dipropionate ester, clobetasol or esters thereof, such as the propionate, hydrocortisone or esters thereof, such as the acetate; non-steroidal anti-inflammatory drugs such as naproxen, indomethacin, diclofenac, ibuprofen, dexibuprofen, ketoprofen, flurbiprofen, piroxicam, lornoxicam or nabumeton, phosphodiesterase 4 inhibitors or p38 MAP kinase inhibitors.

40. A composition according to any one of claims 22-39 for use in the treatment of a dermal disease or condition.

41. The composition for use according to claim 40, wherein the dermal disease or condition is psoriasis, sebopsoriasis, pustulosis palmoplantaris, dermatitis, ichtyosis, rosacea or acne.

## Patentansprüche

1. Suspension von Calcipotriol-Monohydrat in Form von Nanokristallen mit einer Partikelgrößenverteilung in einem Bereich von 200-600 nm, bestimmt durch dynamische Lichtstreuung, wobei die Suspension weiter eine wässrige Phase umfasst, beinhaltend ein nicht-ionisches, polymeres Tensid in einer Menge, die ausreicht, um die Bildung von Aggregaten und/oder das Kristallwachstum der Calcipotriol-Monohydrat-Nanokristalle zu verhindern.

2. Suspension nach Anspruch 1, **dadurch gekennzeichnet, dass** das Tensid ausgewählt ist unter Poloxamer- oder Polysorbat-Tensiden und Polyoxyethylen-C₆₋₂₄-Alkylethern.

3. Suspension nach Anspruch 2, **dadurch gekennzeichnet, dass** das Poloxamer ausgewählt ist unter Poloxamer 124, Poloxamer 188, Poloxamer 237, Poloxamer 338 und Poloxamer 407.

4. Suspension nach Anspruch 3, **dadurch gekennzeichnet, dass** das Tensid Poloxamer 188 ist.

5. Suspension nach Anspruch 2, **dadurch gekennzeichnet, dass** das Polysorbat ausgewählt ist unter Polysorbat 20, Polysorbat 40, Polysorbat 60, Polysorbat 61, Polysorbat 80 und Polysorbat 81.

6. Suspension nach Anspruch 2, **dadurch gekennzeichnet, dass** der Polyoxyethylen-C₆₋₂₄-Alkylether Cetomacrogol 1000 ist.

7. Suspension nach einem der Ansprüche 2-6, **dadurch gekennzeichnet, dass** die Menge an Tensid in der wässrigen Phase in einem Bereich von etwa 0,6% bis etwa 1,2% bezogen auf das Gewicht der Suspension liegt.

8. Suspension nach Anspruch 1, **dadurch gekennzeichnet, dass** die Calcipotriol-Monohydrat-Nanokristalle eine mittlere Partikelgröße von 200-350 nm, 350-400 nm oder 400-500 nm, bestimmt durch dynamische Lichtstreuung, aufweisen.

9. Verfahren zur Herstellung von Calcipotriol-Monohydrat-Nanokristallen mit einer Partikelgrößenverteilung in einem Bereich von 200-600 nm, bestimmt durch dynamische Lichtstreung, wobei das Verfahren es umfasst, dass man
(a) das kristalline Calcipotriol-Monohydrat in einer wässrigen Phase, die ein nicht-ionisches, polymeres Tensid in einer Menge von etwa 1% bis etwa 5% bezogen auf das Gewicht der wässrigen Phase umfasst, zerkleinert, wobei Mikropartikel mit einer Partikelgrößenverteilung von etwa 5-20 µm und einer mittleren Teilchengröße von etwa 10 µm gebildet werden;
(b) die Suspension aus Schritt (a) einem ersten Zyklus einer Hochdruckhomogenisierung bei einem Druck von etwa 300-800 bar solange unterzieht bis etwa 15-40% der Calcipotriol-Monohydrat-Kristalle mit einer Partikelgrößenverteilung in einem Bereich von 200-600 nm erhalten werden;
(c) die Suspension aus Schritt (b) einem zweiten Zyklus einer Hochdruckhomogenisierung bei einem Druck von etwa 800-1200 bar solange unterzieht bis etwa 40-80% der Calcipotriol-Monohydrat-Kristalle mit einer Partikelgrößenverteilung in einem Bereich von 200-600 nm erhalten werden;
(d) die Suspension aus Schritt (c) einem dritten Zyklus einer Hochdruckhomogenisierung bei einem Druck von etwa 1200-1700 bar solange unterzieht bis etwa 90% oder mehr der Calcipotriol-Monohydrat-Kristalle mit einer Partikelgrößenverteilung in einem Bereich von 200-600 nm erhalten werden;
und
(e) gegebenenfalls die erhaltenen Calcipotriol-Monohydrat-Nanokristalle aus der wässrigen Phase isoliert.

10. Verfahren nach Anspruch 9, wobei das Verkleinern in Schritt (a) mit Hilfe einer Nass-Kugelmühle unter Verwendung von Kugeln oder Perlen mit einem Durchmesser im Bereich von 1-4 mm, zum Beispiel 1,5-2,5 mm oder 2-3 mm, durchgeführt wird.

11. Verfahren nach Anspruch 9, wobei das in Schritt (a) verwendete Tensid ausgewählt ist unter Poloxamer- oder Polysorbat-Tensiden und Polyoxyethylen-C₆₋₂₄-Alkylethern.

12. Verfahren nach Anspruch 11, wobei das Poloxamer ausgewählt ist unter Poloxamer 124, Poloxamer 188, Poloxamer 237, Poloxamer 338 und Poloxamer 407.

13. Verfahren nach Anspruch 12, wobei das Tensid Poloxamer 188 ist.

14. Verfahren nach Anspruch 11, wobei das Polysorbat ausgewählt ist unter Polysorbat 20, Polysorbat 40, Polysorbat 60, Polysorbat 61, Polysorbat 80 und Polysorbat 81.

15. Verfahren nach Anspruch 11, wobei der Polyoxyethylen-C₆₋₂₄-Alkylether Cetomacrogol 1000 ist.

16. Verfahren nach einem der Ansprüche 9-15, wobei das Tensid in Schritt (a) in einer Menge von etwa 1,5 bis etwa 3%, bezogen auf das Gewicht der Suspension, zugegeben wird, insbesondere in einer Menge von etwa 2%, bezogen auf das Gewicht der Suspension.

17. Verfahren nach Anspruch 9, wobei der erste Zyklus der Hochdruckhomogenisierung in Schritt (b) bei einem Druck von etwa 500-650 bar durchgeführt wird.

18. Verfahren nach Anspruch 9, wobei der zweite Zyklus der Hochdruckhomogenisierung in Schritt (c) bei einem Druck von etwa 1000-1100 bar durchgeführt wird.

19. Verfahren nach Anspruch 9, wobei der dritte Zyklus der Hochdruckhomogenisierung in Schritt (d) bei einem Druck von etwa 1400-1500 bar durchgeführt wird.

20. Verfahren nach einem der Ansprüche 9-19, wobei die Hochdruckhomogenisierungsschritte (b)-(d) unter Verwendung eines Kolbenspalthomogenisierers durchgeführt werden.

21. Verfahren nach einem der Ansprüche 9-20, weiter umfassend Gefriertrocknen und Sprühtrocknen der Calcipotriol-Monohydrat-Nanokristalle.

22. Pharmazeutische Zusammensetzung umfassend die Suspension von Caclipotriol-Monohydrat-Nanokristallen eines der Ansprüche 1-8 und einen pharmazeutisch annehmbaren Träger.

23. Zusammensetzung nach Anspruch 22, **dadurch gekennzeichnet, dass** die Menge an nicht-ionischem, polymerem Tensid in einem Bereich von etwa 0,03-0,06% bezogen auf das Gewicht der Zusammensetzung liegt.

24. Zusammensetzung nach Anspruch 22, **dadurch gekennzeichnet, dass** der Träger mindestens ein Paraffin umfasst, ausgewählt unter Paraffinen bestehend aus Kohlenwasserstoffen mit Kettenlängen von C₅₋₆₀, wobei die Kettenlängen C₁₄₋₁₆, C₁₈₋₂₂, C₂₀₋₂₂, C₂₀₋₂₆, C₂₈₋₄₀ und C₄₀₋₄₄ oder Mischungen davon am häufigsten vertreten sind.

25. Zusammensetzung nach einem der Ansprüche 22-24, weiter umfassend einen Wasserin-ÖI-Emulgator ausgewählt unter Polyoxyethylen-C₈₋₂₂-Alkylethern, zum Beispiel Polyoxyethylenstearylether, Polyoxyethylencetylether oder Polyoxyethylenlaurylether.

26. Zusammensetzung nach einem der Ansprüche 22-25, weiter umfassend einen lipophilen Bestandteil zur Erhöhung der Viskosität.

27. Zusammensetzung nach Anspruch 26, **dadurch gekennzeichnet, dass** der Bestandteil zur Erhöhung der Viskosität ein Wachs ist.

28. Zusammensetzung nach einem der Ansprüche 22-27, weiter umfassend ein Silikonwachs oder ein flüchtiges Silikonöl.

29. Zusammensetzung nach Anspruch 28, **dadurch gekennzeichnet, dass** das flüchtige Silikonöl Cyclomethicon oder -dimethicon ist.

30. Zusammensetzung nach einem der Ansprüche 22-29, weiter umfassend eine antiirritative Verbindung.

31. Zusammensetzung nach Anspruch 30, **dadurch gekennzeichnet, dass** die antiirritative Verbindung Glycerol, Sorbitol, Sucrose, Saccharin, Nikotinamid, Menthol oder Eukalyptol ist.

32. Zusammensetzung nach einem der Ansprüche 22-31, weiter umfassend eine Verbindung, die in der Lage ist, saure Verunreinigungen, die schädlich für die chemische Stabilität von Calcipotriol-Monohydrat in der Zusammensetzung sind, zu neutralisieren.

33. Zusammensetzung nach Anspruch 32, **dadurch gekennzeichnet, dass** diese Verbindung ein Amin, wie zum Beispiel Triethanolamin, Trometamol, Monoethanolamin oder Dimethanolamin ist.

34. Zusammensetzung nach einem der Ansprüche 22-33, die eine Salbe ist.

35. Zusammensetzung nach Anspruch 34, die eine im Wesentlichen wasserfreie Salbe ist.

36. Zusammensetzung nach einem der Ansprüche 22-33, die eine Creme ist.

37. Zusammensetzung nach einem der Ansprüche 22-36, umfassend 0,001-0,5 mg/g, vorzugsweise etwa 0,002-0,25 mg/g, insbesondere 0,005-0,5 mg/g, an Calcipotriol-Monohydrat-Nanokristallen.

38. Zusammensetzung nach einem der Ansprüche 22-37, weiter umfassend einen oder mehrere weitere therapeutisch aktive Bestandteile.

39. Zusammensetzung nach Anspruch 38, **dadurch gekennzeichnet, dass** die zusätzlichen aktiven Bestandteile ausgewählt sind unter entzündungshemmenden Arzneimitteln wie zum Beispiel Corticosteroiden, wie zum Beispiel Betamethason und Estern davon, zum Beispiel dem Valeratester oder Dipropionatester, Clobetasol und Estern davon, zum Beispiel dem Propionat, Hydrocortison oder Estern davon, zum Beispiel dem Acetat; nichtsteroidalen entzündungshemmenden Arzneimitteln wie zum Beispiel Naproxen, Indomethacin, Diclofenac, Ibuprofen, Dexibuprofen, Ketoprofen, Flurbiprofen, Piroxicam, Lornoxicam oder Nabumeton, Phosphodiesterase-4-inhibitoren oder p38 MAP Kinaseinhibitoren.

40. Zusammensetzung nach einem der Ansprüche 22-39 zur Verwendung in der Behandlung von Hautkrankheiten oder -erkrankungen.

41. Die Zusammensetzung zur Verwendung nach Anspruch 40, **dadurch gekennzeichnet, dass** die Hautkrankheit oder -erkrankung Psoriasis, Sebopsoriasis, Pustulosis Palmoplantaris, Dermatitis, Ichtoysis, Rosacea oder Akne ist.

## Revendications

1. Suspension de calcipotriol monohydraté sous forme de nanocristaux d'une distribution de taille de particule dans la plage de 200-600 nm telle qu'elle est déterminée par diffusion de la lumière dynamique, la suspension comprenant en outre une phase aqueuse incluant un tensioactif polymère non ionique en une quantité suffisante pour empêcher la formation d'agrégats et/ou la croissance cristalline des nanocristaux de calcipotriol monohydraté.

2. Suspension selon la revendication 1, où le tensioactif est choisi dans le groupe consistant en les tensioactifs poloxamères ou polysorbates, et un polyoxyéthylène C₆₋₂₄ alkyl éther.

3. Suspension selon la revendication 2, où le poloxamère est choisi dans le groupe consistant en poloxamère 124, poloxamère 188, poloxamère 237, poloxamère 338 et poloxamère 407.

4. Suspension selon la revendication 3, où le tensioactif est le poloxamère 188.

5. Suspension selon la revendication 2, où le polysorbate est choisi dans le groupe consistant en polysorbate 20, polysorbate 40, polysorbate 60, polysorbate 61, polysorbate 80 et polysorbate 81.

6. Suspension selon la revendication 2, où le polyoxyéthylène C₆₋₂₄ alkyléther est le cétomacrogol 1000.

7. Suspension selon l'une quelconque des revendications 2-6, où la quantité de tensioactif dans ladite phase aqueuse est dans la plage d'environ 0,6 % à environ 1,2 % en poids de la suspension.

8. Suspension selon la revendication 1, où les nanocristaux de calcipotriol monohydraté ont une taille de particule moyenne de 200-350 nm, 350-400 nm ou 400-500 nm telle qu'elle est déterminée par diffusion de la lumière dynamique.

9. Procédé pour préparer des nanocristaux de calcipotriol monohydraté d'une distribution de taille de particule dans la plage de 200-600 nm telle qu'elle est déterminée par diffusion de la lumière dynamique, le procédé comprenant les étapes de
(a) diminution du calcipotriol monohydraté cristallin dans une phase aqueuse comprenant un tensioactif polymère non ionique en une quantité dans la plage d'environ 1 % à environ 5 % en poids de ladite phase aqueuse, conduisant à la formation de microparticules ayant une distribution de taille de particule dans la plage d'environ 5-20 µm et une taille de particule moyenne d'environ 10 µm ;
(b) exposition de la suspension de l'étape (a) à un premier cycle d'homogénéisation haute pression à une pression d'environ 300-800 bar pendant une durée suffisante pour obtenir environ 15-40 % de cristaux de calcipotriol monohydraté ayant une distribution de taille de particule dans la plage de 200-600 nm ;
(c) exposition de la suspension de l'étape (b) à un second cycle d'homogénéisation haute pression à une pression d'environ 800-1200 bar une durée suffisante pour obtenir environ 40-80 % de cristaux de calcipotriol monohydraté ayant une distribution de taille de particule dans la plage de 200-600 nm ;
(d) exposition de la suspension de l'étape (c) à un troisième cycle d'homogénéisation haute pression à une pression d'environ 1200-1700 bar une durée suffisante pour obtenir environ 90 % ou plus de cristaux de calcipotriol monohydraté ayant une distribution de taille de particule dans la plage de 200-600 nm ;
et
(e) éventuellement isolement des nanocristaux de calcipotriol monohydraté résultants à partir de la phase aqueuse.

10. Procédé selon la revendication 9, où la diminution de l'étape (a) est conduite par broyage à boulets humide utilisant des boulets ou des billes d'un diamètre dans la plage de 1-4 mm, comme 1,5-2,5 mm ou 2-3 mm.

11. Procédé selon la revendication 9, où le tensioactif utilisé dans l'étape (a) est choisi dans le groupe consistant en les tensioactifs poloxamères ou polysorbates, et un polyoxyéthylène C₆₋₂₄ alkyléther.

12. Procédé selon la revendication 11, où le poloxamère est choisi dans le groupe consistant en poloxamère 124, poloxamère 188, poloxamère 237, poloxamère 338 et poloxamère 407.

13. Procédé selon la revendication 12, où le tensioactif est le poloxamère 188.

14. Procédé selon la revendication 11, où le polysorbate est choisi dans le groupe consistant en polysorbate 20, polysorbate 40, polysorbate 60, polysorbate 61, polysorbate 80 et polysorbate 81.

15. Procédé selon la revendication 11, où le polyoxyéthylène C₆₋₂₄ alkyléther est le cétomacrogol 1000.

16. Procédé selon l'une quelconque des revendications 9-15, où le tensioactif est ajouté dans l'étape (a) en une quantité dans la plage d'environ 1,5 à environ 3 % en poids de la suspension, en particulier environ 2 % en poids de la suspension.

17. Procédé selon la revendication 9, où le premier cycle d'homogénéisation haute pression de l'étape (b) est réalisé à une pression d'environ 500-650 bar.

18. Procédé selon la revendication 9, où le second cycle d'homogénéisation haute pression de l'étape (c) est réalisé à une pression d'environ 1000-1100 bar.

19. Procédé selon la revendication 9, où le troisième cycle d'homogénéisation haute pression de l'étape (d) est réalisé à une pression d'environ 1400-1500 bar.

20. Procédé selon l'une quelconque des revendications 9-19, où les étapes d'homogénéisation haute pression (b)-(d) sont réalisées au moyen d'un homogénéisateur à piston et interstice.

21. Procédé selon l'une quelconque des revendications 9-20 comprenant en outre la lyophilisation ou le séchage par pulvérisation des nanocristaux de calcipotriol monohydraté.

22. Composition pharmaceutique comprenant la suspension de nanocristaux de calcipotriol monohydraté selon l'une quelconque des revendications 1-8 et un vecteur pharmaceutiquement acceptable.

23. Composition selon la revendication 22, où la quantité de tensioactif polymère non ionique est dans la plage d'environ 0,03-0,06 % en poids de la composition.

24. Composition selon la revendication 22, où le vecteur comprend au moins une paraffine choisie parmi les paraffines consistant en hydrocarbures ayant des longueurs de chaîne de C₅₋₆₀, les longueurs de chaîne atteignant un maximum à C₁₄₋₁₆, C₁₈₋₂₂, C₂₀₋₂₂, C₂₀₋₂₆, C₂₈₋₄₀ et C₄₀₋₄₄, ou leurs mélanges.

25. Composition selon l'une quelconque des revendications 22-24, comprenant en outre un émulsifiant eau-dans-huile choisi parmi les polyoxyéthylène C₈₋₂₂ alkyléthers, par exemple polyoxyéthylène stéaryléther, polyoxyéthylène cétyléther ou polyoxyéthylène lauryléther.

26. Composition selon l'une quelconque des revendications 22-25, comprenant en outre un ingrédient augmentant la viscosité lipophile.

27. Composition selon la revendication 26, où l'ingrédient augmentant la viscosité est une cire.

28. Composition selon l'une quelconque des revendications 22-27, comprenant en outre une cire de silicone ou une huile de silicone volatile.

29. Composition selon la revendication 28, où l'huile de silicone volatile est le cyclométhicone ou le diméthicone.

30. Composition selon l'une quelconque des revendications 22-29, comprenant en outre un composé anti-irritation.

31. Composition selon la revendication 30, où le composé anti-irritation est le glycérol, le sorbitol, le saccharose, la saccharine, le nicotinamide, le menthol ou l'eucalyptol.

32. Composition selon l'une quelconque des revendications 22-31, comprenant en outre un composé capable de neutraliser les impuretés acides néfastes pour la stabilité chimique du calcipotriol monohydraté dans la composition.

33. Composition selon la revendication 32, où ledit composé est une amine comme la triéthanolamine, le trométamol, la monoéthanolamine ou la diméthanolamine.

34. Composition selon l'une quelconque des revendications 22-33 qui est une pommade.

35. Composition selon la revendication 34 qui est une pommade sensiblement anhydre.

36. Composition selon l'une quelconque des revendications 22-33 qui est une crème.

37. Composition selon l'une quelconque des revendications 22-36 comprenant environ 0,001-0,5 mg/g, de préférence environ 0,002-0,25 mg/g, en particulier 0,005-0,05 mg/g, de nanocristaux de calcipotriol monohydraté.

38. Composition selon l'une quelconque des revendications 22-37, comprenant en outre un ou plusieurs ingrédients thérapeutiquement actifs supplémentaires.

39. Composition selon la revendication 38, où de tels ingrédients actifs supplémentaires sont choisis dans le groupe consistant en les médicaments anti-inflammatoires comme les corticostéroïdes, comme la bétaméthasone et ses esters, par exemple l'ester valérate ou dipropionate, le clobétasol ou ses esters, comme le propionate, l'hydrocortisone ou ses esters, comme l'acétate ; les médicaments anti-inflammatoires non stéroïdiens comme naproxène, indométhacine, diclofénac, ibuprofène, dexibuprofène, kétoprofène, fluriprofène, piroxicam, lornoxicam ou nabuméton, les inhibiteurs de phosphodiestérase 4 ou les inhibiteurs de MAP kinase p38.

40. Composition selon l'une quelconque des revendications 22-39 destinée à être utilisée dans le traitement d'une maladie ou affection dermique.

41. Composition destinée à être utilisée selon la revendication 40, où la maladie ou affection dermique est le psoriasis, le sébopsoriasis, la pustulose palmoplantaire, la dermatite, l'ichtyose, l'acné rosacée ou l'acné.
